# EUROPÄISCHE PATENTSCHRIFT

(11) **EP 2 190 805 B1**
(45) Veröffentlichungstag und Bekanntmachung des Hinweises auf die Patenterteilung: **17.02.2016**
(21) Anmeldenummer: 08786741.2
(22) Anmeldetag: 01.08.2008
(51) Int. Cl.: C07C 51/235, C07C 53/124, C07C 53/126, C07C 53/128, B01J 8/22

(54) **VERFAHREN UND VORRICHTUNG ZUR OXIDATION VON ALDEHYDEN**
PROCESS AND DEVICE FOR THE OXIDATION OF ALDEHYDES
PROCEDE ET DISPOSITIF D'OXYDATION DES ALDEHYDES

(30) Priorität: 21.08.2007 EP 07114711
(43) Veröffentlichungstag der Anmeldung: 02.06.2010
(73) Patentinhaber: BASF SE, 67056 Ludwigshafen (DE)
(72) Erfinder: TELES, Joaquim Henrique, 67166 Otterstadt (DE); GUMLICH, Kai, 68159 Mannheim (DE); SCHÄFER, Jochen, 90402 Nürnberg (DE); OEHLENSCHLÄGER, Steffen, 67063 Ludwigshafen (DE); LAMM, Stephan, 68723 Oftersheim (DE); MERTEN, Roland, 69469 Weinheim (DE); SCHÄFER, Martin, 67269 Grünstadt (DE); GROB, Rüdiger, 67686 Mackenbach (DE); EMNET, Uwe, 67363 Lustadt (DE)
(86) Internationale Anmeldenummer: PCT/EP2008/060121
(87) Internationale Veröffentlichungsnummer: WO 2009/024446

(56) Entgegenhaltungen:
- EP-A- 0 733 401
- DE-A1- 19 835 907
- DE-A1- 19 854 637
- DE-A1-102004 021 763
- US-A1- 2006 047 147

## Beschreibung

Die Erfindung betrifft ein Verfahren zur Oxidation von Aldehyden mit Sauerstoff. Weiterhin umfasst die Erfindung einen Reaktor zur Durchführung des Verfahrens.

Das Verfahren eignet sich zum Beispiel zur Oxidation von Aldehyden mit Sauerstoff zu ihren korrespondierenden Säuren.

Die Herstellung organischer Säuren ist zum Beispiel aus WO 99/54274 bekannt. Hierzu werden eine oder mehrere organische Flüssigkeiten mit im Wesentlichen reinem Sauerstoff oder sauerstoffangereicherter Luft, die mindestens 50 % Sauerstoff enthält, in einem Flüssigoxidationsreaktor oxidiert. Die Temperatur wird innerhalb eines Bereichs von ± 3°C um eine Zieltemperatur gehalten. Nach der Reaktion wird das Reaktionsgemisch aufbereitet. Die Aufbereitung erfolgt zum Beispiel durch Destillation, Lösungsmittelextraktion, Kristallisation, Verdampfung, Phasentrennung, Filtration oder ähnliches. Zur Reaktion wird ein Schlaufenreaktor eingesetzt, bei dem im Reaktor ein Einsteckrohr enthalten ist. Im Raum zwischen dem Einsteckrohr und der Kolonnenwand ist ein Wärmetauscher aufgenommen. Durch einen Rührer im Einsteckrohr wird die Flüssigkeitsströmung im Reaktor erzeugt.

Ein Schlaufenreaktor zur Durchführung von Gas-Flüssig-, Flüssig-Flüssig- oder Gas-Flüssig-Fest-Reaktionen ist aus WO-A 00/30743 bekannt. Der Reaktor umfasst eine im oberen Reaktorbereich angeordnete, nach unten gerichtete Strahldüse, über die Edukte und das Reaktionsgemisch zugeführt werden, sowie einen Abzug, bevorzugt im unteren Reaktorbereich, über den das Reaktionsgemisch in einen äußeren Kreislauf mittels einer Pumpe der Strahldüse erneut zugeführt wird. Im Reaktor ist ein konzentrisches Leitrohr angeordnet, das sich im Wesentlichen über die gesamte Länge des Reaktors mit Ausnahme der Reaktorenden erstreckt. Das Leitrohr weist eine Querschnittsfläche im Bereich von 1/10 bis zur Hälfte der Querschnittsfläche des Reaktors auf. Die Strahldüse ist oberhalb des oberen Endes des Leitrohrs, bevorzugt um 1/8 des Leitrohrdurchmessers bis zu einem Leitrohrdurchmesser beabstandet, angeordnet oder taucht in das Leitrohr in eine Tiefe bis zu mehreren Leitrohrdurchmessern ein. In den Ringraum ist ein Wärmetauscher integriert. Der Reaktor wird zum Beispiel zur Herstellung von Propionsäure aus Propionaldehyd eingesetzt. Hierzu wird das Propionaldehyd mit Sauerstoff aus Luft oxidiert. Um einen erhöhten Umsatz zu erzielen ist beschrieben, mehrere der Reaktoren in Reihe zu schalten.

Aus WO 01/66505 ist ein Verfahren zur Herstellung aliphatischer Carbonsäuren mit 4 bis 10 Kohlenstoffatomen durch Oxidation der entsprechenden Aldehyde mit Sauerstoff oder Sauerstoff enthaltenden Gasen bekannt. Die Oxidation wird im Temperaturbereich von 0 bis 100°C in mindestens zwei Stufen bei von Stufe zu Stufe ansteigenden Temperaturen durchgeführt. Für jede Reaktionsstufe ist ein Reaktor vorgesehen. Als Reaktor werden zum Beispiel Rohrreaktoren, die gegebenenfalls noch Füllkörper enthalten, Rieseltürme, die Füllkörper enthalten, oder Blasensäulen beschrieben.

Ebenfalls ein Verfahren zur Oxidation einer organischen Substanz, bei der Luft, Sauerstoff, sauerstoffangereicherte oder Sauerstoff enthaltende Luft in eine Flüssigkeit in einem Reaktionssystem geleitet wird, ist aus WO 01/46111 bekannt. Die Reaktion wird bei einer Temperatur im Bereich zwischen 20 und 100°C und einem Druck im Bereich zwischen 0 und 3 bar durchgeführt. Im Reaktionssystem sind Mittel vorgesehen, um ein Durchmischen der Flüssigkeitsmenge zu erzielen. Zum Erreichen der Durchmischung können zum Beispiel Rührer, Flügelräder, Turbinen, Injektoren, eingetauchte poröse Diffuser, Einblasrohre oder Oberflächenperlatoren eingesetzt werden. Im Reaktor können Umlenkbleche aufgenommen sein.

US 2006/047147 offenbart ein Verfahren und eine Vorrichtung zur verbesserten Durchführung von Flüssigphasenoxidationen. DE 198 35 907 und DE 10 2004 021 763 beschreiben mehrstufige Verfahren zur Umsetzung einer organischen Verbindung mit einem Hydroperoxid beziehungsweise zum Langzeitbetrieb einer heterogen katalysierten Gasphasenpartialoxidation von Acrolein zu Acrylsäure. Ein weiterer mehrstufiger Prozess ist in EP-A 0 733 401 beschrieben, wobei zur Durchführung von Gas/Flüssigreaktionen eine Reaktorkaskade aus zwei Strahldüsenreaktoren und einem Verweilzeitbehälter eingesetzt wird.

Nachteil der aus dem Stand der Technik bekannten Verfahren ist zum einen, dass einige von diesen nur als Batch-Verfahren oder Semi-Batch-Verfahren betrieben werden können. Ein weiterer Nachteil ist, dass die bekannten Verfahren teilweise komplizierte - und daher teure - Apparate verwenden. Zudem benötigt ein Teil der aus dem Stand der Technik bekannten Verfahren mehr als einen Reaktor, um die gewünschten hohen Umsätze zu erzielen.

Aufgabe der vorliegenden Erfindung ist es, ein kontinuierliches Verfahren zur Oxidation von Aldehyden bereitzustellen. Eine weitere Aufgabe der vorliegenden Erfindung ist es, einen Reaktor bereitzustellen, in dem eine Oxidation von Aldehyden mit Sauerstoff durchgeführt werden kann und der auch bei Bildung von zündfähigen Gasgemischen sicher betrieben werden kann.

Gelöst wird die Aufgabe durch ein Verfahren zur Oxidation von Aldehyden mit Sauerstoff, welches folgende Schritte aufweist:
(a) Zugabe des Aldehyds und zumindest eines Teils des für die Oxidation benötigten Sauerstoffs in eine erste Reaktionszone, wobei die erste Reaktionszone isotherm betrieben wird,
(b) Zufuhr mindestens eines Teils des Reaktionsgemisches aus der ersten Reaktionszone in eine zweite Reaktionszone, wobei die zweite Reaktionszone adiabat betrieben wird
und wobei alle Reaktionszonen in einer gemeinsamen Reaktorhülle aufgenommen sind.

Das erfindungsgemäße Verfahren eignet sich zur Oxidation von Aldehyden mit Sauerstoff oder einem sauerstoffhaltigen Gemisch, wobei der Sauerstoffanteil im Gemisch größer als 50 Vol.-% ist.

Das Verfahren eignet sich jedoch insbesondere zur Oxidation von aliphatischen Aldehyden mit Sauerstoff unter Bildung von Carbonsäuren.

Vorteil des erfindungsgemäßen Verfahrens ist es, dass der aliphatische Aldehyd bei Oxidation mit Sauerstoff im Wesentlichen vollständig umgesetzt wird. Im Wesentlichen vollständig bedeutet dabei, dass der Umsatz größer als 97 %, bevorzugt größer als 98 % ist.

Der eingesetzte Aldehyd ist dabei vorzugsweise ein Aldehyd der Formel

R-CHO.

Darin bedeutet R ein C₁-C₂₅-Alkyl, bevorzugt ein C₂-C₂₀-Alkyl. Das Alkyl kann verzweigt oder unverzweigt sein.

Bevorzugt ist das verzeigte oder unverzweigte C₁-C₂₅-Alkyl ausgewählt aus Ethyl, Propyl, Isopropyl, n-Butyl, sec.-Butyl, iso-Butyl, Pent-2-yl, Pent-3-yl, n-Pentyl, n-Hexyl, Hept-2-yl, Hept-3-yl, Oct-4-yl, 2-Methyl-4,4-dimethylpentyl, 2,6-Dimethylheptyl, Non-4-yl, 3-Methyloct-5-yl.

In einer bevorzugten Verfahrensvariante ist zwischen der ersten Reaktionszone, die isotherm und rückvermischt betrieben wird, und der zweiten, adiabat betriebenen Reaktionszone mindestens eine weitere isotherm und rückvermischt betriebene Reaktionszone ausgebildet. Zur Durchführung der Reaktion durchläuft das Reaktionsgemisch nacheinander die einzelnen Reaktionszonen. Durch die Rückvermischung wird innerhalb jeder isotherm betriebenen Reaktionszone eine weitgehend homogene Konzentrationsverteilung innerhalb dieser Reaktionszone erreicht.

Wenn mehrere isotherm und rückvermischt betriebene Reaktionszonen eingesetzt werden, ist es bevorzugt, dass mehreren der isotherm und rückvermischt betriebenen Reaktionszonen Sauerstoff zugegeben wird. So wird der ersten Reaktionszone ein Teil des Sauerstoffs zugegeben, wodurch ein Teil des Aldehyds umgesetzt wird. Ein weiterer Teil des benötigten Sauerstoffs wird dann jeweils den weiteren isotherm und rückvermischt betriebenen Reaktionszonen zugegeben. In diesen werden dann weitere Teile des enthaltenen Aldehyds umgesetzt. Es ist möglich, jeder der isotherm und rückvermischt betriebenen Reaktionszonen ein Teil des Sauerstoffes zuzuführen. Weiterhin ist es jedoch auch möglich, dass bei mehreren isotherm und rückvermischt betriebenen Reaktionszonen nur einem Teil dieser Reaktionszonen Sauerstoff zugeführt wird.

Der zur Reaktion benötigte Sauerstoff kann zum Beispiel in Form eines sauerstoffhaltigen Gases oder als reiner Sauerstoff zugeführt werden. Wenn der Sauerstoff in Form eines sauerstoffhaltigen Gasgemisches eingesetzt wird, enthält dieses vorzugsweise mindestens 50 Vol.-% Sauerstoff, mehr bevorzugt mindestens 75 Vol.-% Sauerstoff, besonders bevorzugt mindestens 90 Vol.-% Sauerstoff. Ganz besonders bevorzugt wird jedoch Sauerstoff mit einer Reinheit von mehr als 99 Vol.-% eingesetzt. Dieser wird auch als reiner Sauerstoff bezeichnet. Wenn ein Gasgemisch eingesetzt wird, so enthält dieses neben dem Sauerstoff vorzugsweise für die Reaktion inerte Gase. Insbesondere enthält das Gasgemisch neben Sauerstoff auch Stickstoff. Das Gasgemisch kann zum Beispiel sauerstoffangereicherte Luft sein.

In einer besonders bevorzugten Ausführungsform umfasst das Verfahren zur Oxidation von Aldehyden zwei isotherm und rückvermischt betriebene Reaktionszonen. Dabei werden der ersten Reaktionszone vorzugsweise mindestens 60 Vol.-%, mehr bevorzugt mindestens 60 Vol.-% und insbesondere mindestens 80 Vol.-% des insgesamt eingesetzten Sauerstoffes oder sauerstoffhaltigen Gases zugeführt. Der restliche eingesetzte Sauerstoff wird in der zweiten Reaktionsstufe zugegeben.

Wenn das Verfahren mehr als zwei isotherme und rückvermischte Reaktionszonen umfasst und der Sauerstoff oder das sauerstoffhaltige Gas in drei Reaktionsstufen zugegeben wird, wird der ersten Reaktionsstufe ebenfalls vorzugsweise mindestens 60 Vol.-%, mehr bevorzugt mindestens 60 Vol.-% und insbesondere mindestens 80 Vol.-% des insgesamt eingesetzten Sauerstoffes oder sauerstoffhaltigen Gases zugeführt.

Der zweiten Reaktionsstufe wird vorzugsweise soviel Sauerstoff oder sauerstoffhaltiges Gas zugeführt, dass in der dritten Reaktionsstufe maximal 10 Vol.-% des eingesetzten Sauerstoffes oder sauerstoffhaltigen Gases zugegeben werden.

Der Sauerstoff wird vorzugsweise im Überschuss eingesetzt. Dieser Überschuss beträgt vorzugsweise 1 bis 20 mol-%, besonders bevorzugt 2 bis 10 mol-% bezogen auf die theoretisch benötigte Menge.

Die Temperatur, bei welcher die isotherm und rückvermischt betriebenen Reaktionszonen betrieben werden, liegt vorzugsweise im Bereich zwischen 2 und 140°C, mehr bevorzugt zwischen 30 und 100°C. Der Druck am Ausgang der letzten Reaktionszone liegt vorzugsweise im Bereich zwischen 0,5 bis 10 bar, insbesondere zwischen 1 und 5 bar. Hierbei wird vorzugsweise nur eine Druckregelung eingesetzt, die sich am Ausgang der letzten Reaktionszone befindet. Da alle Reaktionszonen miteinander kommunizieren, bestimmt diese Druckregelung den Druck in allen Reaktionszonen. Im Allgemeinen nimmt der Druck von Reaktionszone zu Reaktionszone ab.

Wenn mehrere isotherm und rückvermischt betriebene Reaktionszonen eingesetzt werden, so ist es bevorzugt, wenn die Temperatur in allen isotherm und rückvermischt betriebenen Reaktionszonen gleich ist. Gleich im Sinne der Erfindung bedeutet, dass die Temperaturunterschiede zwischen den mittleren Temperaturen der einzelnen Reaktionszonen nicht größer als 5°C sind.

Alternativ ist es jedoch auch möglich, dass die isotherm und rückvermischt betriebenen Reaktionszonen derart betrieben werden, dass die Temperatur von Reaktionszone zu Reaktionszone zunimmt. Die Temperaturdifferenz zwischen den einzelnen Reaktionszonen liegt dann vorzugsweise bei mindestens 5°C, bevorzugt mindestens 10°C.

Isotherm betrieben im Sinne der vorliegenden Erfindung bedeutet, dass die Temperaturunterschiede innerhalb einer Reaktionszone nicht größer als 10°C sind. Dieser Temperaturunterschied ergibt sich aus dem Wärmetransport zum Wärmetauscher der für den isothermen Betrieb notwendig ist.

Eine vollständige Rückvermischung innerhalb der isotherm und rückvermischt betriebenen Reaktionszonen wird zum Beispiel dadurch erzielt, dass die mindestens eine isotherm und rückvermischt betriebene Reaktionszone nach dem Prinzip eines Strahlschlaufenreaktors arbeitet. Hierzu wird ein Teil des Reaktionsgemisches der Reaktionszone entnommen und über eine Düse im oberen Bereich der Reaktionszone wieder zugeführt. Hierdurch wird eine Ringströmung in der Reaktionszone erzeugt. Die Düse ist dabei vorzugsweise axial in der Reaktionszone angeordnet. In einer bevorzugten Ausführungsform ist in der Reaktionszone ein Einsteckrohr enthalten, welches von dem Reaktionsgemisch umströmt wird. Die bei der Reaktion entstehende Wärme wird mittels eines Wärmetauschers aus dem Reaktionsgemisch abgeführt. Der Wärmetauscher kann dabei innerhalb der Reaktionszone oder außerhalb der Reaktionszone angeordnet sein. Wenn der Wärmetauscher innerhalb der Reaktionszone angeordnet ist, so ist es bevorzugt, wenn zum Beispiel Wärmetauscherrohre zwischen dem Einsteckrohr und der Außenwand der Reaktionszone angeordnet sind. Weiterhin ist es auch möglich, dass die Reaktionszone an ihrer Außenwand temperiert wird. Eine weitere Möglichkeit ist auch, dass das Einsteckrohr von einem Wärmeträger durchströmt wird und so die Reaktionszone temperiert wird.

Bei einem außenliegenden Wärmetauscher ist dieser vorzugsweise so angeordnet, dass der Anteil des Reaktionsgemisches, welcher der Reaktionszone entnommen und über die Düse im oberen Bereich der Reaktionszone wieder zugeführt wird, durch den Wärmetauscher geleitet und so temperiert wird.

Die Düse, mit der das Reaktionsgemisch der Reaktionszone zum Erzeugen der Strömung zugeführt wird ist vorzugsweise eine Zweistoffdüse. Über diese Zweistoffdüse wird zusammen mit dem Reaktionsgemisch auch der für die Reaktion benötigte Sauerstoff zugeführt. Durch den Einsatz der Zweistoffdüse ergibt sich eine Vermischung des Reaktionsgemisches mit dem Sauerstoff oder sauerstoffhaltigen Gas, wobei der Sauerstoff oder das sauerstoffhaltige Gas mit der Strömung mitgerissen wird. Eine gleichmäßige Verteilung des Sauerstoffes im Reaktionsgemisch wird erzielt. Somit wird auch der Aldehyd gleichmäßig umgesetzt.

Die adiabat betriebene Reaktionszone ist vorzugsweise nicht rückvermischt und in Form einer Blasensäule ausgebildet. In die Blasensäule tritt das Reaktionsgemisch aus der letzten isothermen und rückvermischten Reaktionszone ein. Dieses Reaktionsgemisch enthält noch nicht umgesetzten Sauerstoff. Der Anteil des Sauerstoffes im Reaktionsgemisch, welches in die adiabate Reaktionszone eintritt, liegt vorzugsweise unterhalb von 10 Vol.-% des gesamten eingesetzten Sauerstoffes. In der adiabaten Reaktionszone steigt der Sauerstoff in Form von Blasen im Reaktionsgemisch auf. Das Reaktionsgemisch wird durch die aufsteigenden Blasen vermischt. Hierdurch hat das Reaktionsgemisch Kontakt mit dem Sauerstoff und es werden verbleibender Aldehyd mit dem Sauerstoff oxidiert.

Bevorzugt sind in der adiabaten Reaktionszone Einbauten vorgesehen, um eine Rückvermischung innerhalb dieser Zone zu unterdrücken. Als Einbauten eignen sich zum Beispiel Lochbleche, durch welche eine kaskadierte Blasensäule erzeugt wird, weiterhin sind aber auch Packungen oder eine Schüttung geeignet. Eine weitere Aufgabe der Einbauten ist es, eine gleichmäßige Verteilung der Gasblasen zu erzielen. Die adiabat betriebene Reaktionszone dient als Nachreaktor, um hohe Umsätze der organischen Verbindung zu erzielen. Die adiabate Zone ist vorzugsweise gut gegen die Umgebung isoliert, so dass keine Wärme an die Umgebung abgegeben oder von ihr aufgenommen werden kann.

In der erfindungsgemäßen Ausführungsform sind alle Reaktionszonen in einer gemeinsamen Reaktorhülle aufgenommen. Dabei sind die Reaktionszonen vorzugsweise übereinander angeordnet, wobei sich die erste isotherm und rückvermischte Reaktionszone unten befindet und die adiabate Reaktionszone oben. Die einzelnen Reaktionszonen sind vorzugsweise durch Lochbleche voneinander getrennt. Durch die Anordnung wird gewährleistet, dass unverbrauchter Sauerstoff von einer Reaktionszone direkt in die nächste gelangt.

Die Erfindung betrifft weiterhin einen Reaktor zur Durchführung des Verfahrens, umfassend mindestens eine isotherme und eine adiabate Reaktionszone, die in einer Reaktorhülle angeordnet sind, wobei jede isotherme Reaktionszone in Form eines Strahlschlaufenreaktors ausgebildet ist und die adiabate Reaktionszone als Blasensäule ausgebildet ist.

Jede der isothermen Reaktionszonen, die in Form eines Strahlschlaufenreaktors ausgebildet sind, enthält vorzugsweise ein Einsteckrohr. Dieses ist so ausgebildet, dass die Flüssigkeit innerhalb der Reaktionszone das Einsteckrohr umströmen kann. Eine gleichmäßige Umströmung wird dadurch erzielt, dass das Einsteckrohr koaxial zur Reaktorhülle positioniert ist. Am oberen oder am unteren Ende des Einsteckrohres wird das flüssige Reaktionsmedium zugegeben. An der der Zugabestelle gegenüberliegenden Seite des Einsteckrohres ist eine Prallplatte angeordnet. Die Flüssigkeit tritt durch die Zugabevorrichtung in das Einsteckrohr ein, durchströmt dieses und prallt gegen die Prallplatte. Durch die Prallplatte wird die Flüssigkeit umgelenkt und strömt an der Außenseite des Einsteckrohres in dem zwischen Einsteckrohr und der Reaktorhülle gebildeten Annulusraum in die entgegengesetzte Richtung wieder zurück. Eine Schlaufenströmung entsteht. Aus der ersten Reaktionszone tritt die Flüssigkeit in die nächste Reaktionszone ein. In einer bevorzugten Ausführungsform sind in der Reaktorhülle zwei isotherme Reaktionszonen enthalten, die jeweils in Form eines Strahlschlaufenreaktors mit Einsteckrohr und Prallplatte ausgebildet sind. Die beiden in Form des Strahlschlaufenreaktors ausgebildeten isothermen Reaktionszonen sind in der gemeinsamen Reaktorhülle übereinander angeordnet.

Um die isothermen Reaktionszonen als Strahlschlaufenreaktor betreiben zu können, weisen diese vorzugsweise einen äußeren Flüssigkeitsumlauf auf. Hierzu ist eine Flüssigkeitsentnahmestelle vorzugsweise im unteren Bereich der Reaktionszone vorgesehen und eine Flüssigkeitszugabevorrichtung im oberen Bereich der Reaktionszone. Die Flüssigkeitszugabevorrichtung ist vorzugsweise eine Düse, die zentral oberhalb des Einsteckrohres in der Reaktionszone angeordnet ist. Im Flüssigkeitsumlauf ist vorzugsweise eine Pumpe vorgesehen, mit welcher Flüssigkeit aus der Reaktionszone angesaugt wird. Diese Flüssigkeit wird dann über die Düse wieder in die Reaktionszone zurückgeführt. Die Flüssigkeit wird unter erhöhtem Druck und vorzugsweise mit hoher Geschwindigkeit in die Reaktionszone eingespritzt, damit eine Ringströmung entsteht. Vorzugsweise ist die Strömungsgeschwindigkeit der Ringströmung so groß, dass Gasblasen mit der Strömung mitgerissen werden.

Zur Temperierung des Reaktionsmediums enthält der Flüssigkeitsumlauf vorzugsweise weiterhin mindestens einen Wärmetauscher. Im Wärmetauscher kann das durchlaufende Reaktionsmedium temperiert werden. Die Temperatur in der Reaktionszone kann so eingestellt werden.

In einer bevorzugten Ausführungsform ist die Düse, mit der die Flüssigkeit aus dem Flüssigkeitsumlauf der Reaktionszone zugeführt wird, eine Zweistoffdüse, über welche die Flüssigkeit und Sauerstoff der Reaktionszone zugegeben werden können. Bei einem Reaktor mit zwei isotherm betriebenen Reaktionszonen werden vorzugsweise mindestens 70 Vol.-%, mehr bevorzugt mindestens 80 Vol.-% des eingesetzten Sauerstoffes in der ersten Reaktionszone zugegeben. Der restliche Sauerstoff wird in der zweiten Reaktionszone zugegeben. Wenn noch eine dritte isotherme Reaktionszone vorgesehen ist, so ist die Menge des Sauerstoffes, die in der dritten Reaktionszone zugegeben wird, vorzugsweise kleiner als 10 Vol.-% des eingesetzten Sauerstoffes.

Neben der Ausführungsform, bei der die Düse am oberen Ende des Einsteckrohres angeordnet ist und die Prallplatte am unteren Ende des Einsteckrohres ist es auch möglich, dass die Düse am unteren Ende des Einsteckrohres und die Prallplatte am oberen Ende des Einsteckrohres angeordnet sind. Dies führt dazu, dass das Reaktionsmedium im Einsteckrohr nach oben und um das Einsteckrohr herum nach unten strömt. Bevorzugt ist jedoch die Ausführungsform, bei der die Düse oberhalb des Einsteckrohres und die Prallplatte unterhalb des Einsteckrohres angeordnet sind, so dass die Strömung innerhalb des Einsteckrohres von oben nach unten und außerhalb des Einsteckrohres von unten nach oben erfolgt. Vorteil dieser Ausführungsform ist es, dass die Gasblasen einen längeren Weg zurücklegen müssen, bevor sie in die nachfolgende Reaktionszone strömen und somit eine höhere mittlere Verweilzeit haben. Durch diese höhere Verweilzeit des Gases ist der erreichbare Umsatz bei nach unten gerichteter Düse höher als bei nach oben gerichteter Düse.

Alternativ zu der Ausführungsform, bei der der Wärmetauscher im Flüssigkeitsumlauf aufgenommen ist, ist es auch möglich, dass der Wärmetauscher zum Beispiel im Annulusraum, das heißt zwischen der Kolonnenwand und dem Einsteckrohr, angeordnet ist. Als Wärmetauscher eignen sich zum Beispiel Wärmetauscherohre, die von einem Temperiermedium durchströmt werden. Es kann jedoch jeder beliebige, dem Fachmann bekannte Wärmetauscher eingesetzt werden, mit dem die Temperatur in der Reaktionszone geregelt werden kann.

Auch bei Einsatz eines außenliegenden Wärmetauschers, der im Flüssigkeitsumlauf angeordnet ist, ist jeder dem Fachmann bekannte Wärmetauscher einsetzbar. So können zum Beispiel Rohrbündelwärmetauscher, Plattenwärmetauscher, Spiralwärmetauscher eingesetzt werden.

Der Aldehyd wird vorzugsweise dem Flüssigkeitsumlauf der ersten Reaktionszone zugeführt. Bei mehreren isotherm betriebenen Reaktionszonen ist es jedoch auch möglich, dass ein Teil des Aldehyds dem Flüssigkeitsumlauf der ersten Reaktionszone und weitere Teile den Flüssigkeitsumläufen der weiteren Reaktionszonen zugeführt werden. Hierbei ist es möglich, das jeder Reaktionszone ein Teil des Aldehyds zugeführt wird oder es ist möglich, dass nur einigen der isotherm betriebenen Reaktionszonen der Aldehyd zugeführt wird. Besonders bevorzugt ist es jedoch, dass der Aldehyd, der im Reaktor oxidiert werden soll, nur der ersten Reaktionszone zugeführt wird.

In der adiabaten Reaktionszone sind vorzugsweise Einbauten enthalten. Geeignete Einbauten sind zum Beispiel Lochbleche oder Siebböden, um eine kaskadierte Blasensäule zu erzeugen. Alternativ ist es jedoch auch möglich, dass in der adiabaten Reaktionszone eine Packung oder eine Schüttung enthalten ist.

Um Gas und Flüssigkeit des Reaktionsmediums voneinander zu trennen, schließt sich vorzugsweise an die adiabate Reaktionszone eine weitere Zone an, in der Gas und Flüssigkeit voneinander getrennt werden, wobei sich eine zusammenhängende Gasphase ausbildet. Außerdem wird die Druckauslegung des Reaktors so gewählt, dass dieser eine bei einer Zündung auftretende Druckspitze unbeschadet überstehen kann. Um Folgen einer möglichen Explosion in der Gasphase zu mildern, enthält diese weitere Zone vorzugsweise eine Schüttung oder eine Packung. Die Gasphase enthält im Allgemeinen Sauerstoff, da dieser in Überschuss als Oxidationsmittel eingesetzt wird. Weiterhin können auch Kohlenmonoxid, Kohlendioxid und organische Verbindungen enthalten sein. Aufgrund der gegebenenfalls enthaltenen organischen Verbindungen ist es möglich, dass die Gasphase zündfähig ist.

Als Packung oder Schüttung in der weiteren Zone, die sich der adiabaten Reaktionszone anschließt, kann jede beliebige, dem Fachmann bekannte Schüttung oder Packung eingesetzt werden. So sind zum Beispiel strukturierte Packungen oder Füllkörperschüttungen denkbar. Geeignete strukturierte Packungen oder Füllkörper sind dem Fachmann bekannt und kommerziell erhältlich. Bevorzugt werden Raschig-Ringe oder Pall-Ringe eingesetzt.

Alternativ zu der Ausführungsform, bei der sich eine weitere Zone zur Gas-Flüssigkeits-Phasentrennung an die adiabate Reaktionszone anschließt, ist es auch möglich, dass die Gas-Flüssig-Abscheidung in einem zusätzlichen Apparat erfolgt. Ein geeigneter zusätzlicher Apparat ist zum Beispiel eine Packungs- oder Füllkörperkolonne.

In einer bevorzugten Ausführungsform wird das Reaktionsgemisch abgekühlt, bevor die Gas-Flüssig-Trennung erfolgt. Die Temperatur, auf die das Reaktionsgemisch abgekühlt wird, liegt dabei vorzugsweise unterhalb des Flammpunktes der Flüssigkeit. Hierdurch wird vermieden, dass sich zündfähige Gasgemische bilden.

In einer bevorzugten Ausführungsform wird der Reaktor zur Herstellung einer organischen Säure durch Oxidation des korrespondierenden Aldehyds mit Sauerstoff eingesetzt. Der Aldehyd ist vorzugsweise ausgewählt aus Propionaldehyd, Butyraldehyd, Isobutyraldehyd, Valeraldehyd, 2-Methylbutyraldehyd, 3-Methylbutyraldehyd (Isovaleraldehyd), 2-Ethylbutyraldehyd, n-Hexanal, 2-Methylvaleraldehyd, n-Heptanal, 2-Ethylhexanal, n-Nonanal, 2-Propylheptanal, 2-Propyl-4-methylhexanal, 3,5,5-Trimethylhexanal und 3,7-Dimethyloctanal. Die korrespondierenden Säuren, die durch Oxidation dieser Aldehyde mit Sauerstoff hergestellt werden sind Propionsäure, Buttersäure, Isobuttersäure, Valeriansäure, 2-Methylbuttersäure, Isovaleriansäure, 2-Methylbuttersäure, 3-Methylbuttersäure (Isovaleriansäure), 2-Ethylbuttersäure, n-Hexansäure, 2-Methylvaleriansäure, n-Heptansäure, 2-Ethylhexansäure, n-Nonansäure, 2-Propylheptansäure, 2-Propyl-4-methylhexansäure, 3,5,5-Triemethylhexansäure oder 3,7-Dimethyloctansäure. Es können ebenfalls Gemische aus zwei oder mehr Aldehyden oxidiert werden, zum Beispiel Aldehydgemische, die aus der Hydroformylierung von Olefinen entstehen.

Im Folgenden wird die Erfindung anhand einer Zeichnung näher beschrieben.

Die einzige Figur zeigt schematisch einen erfindungsgemäß ausgebildeten Reaktor mit zwei isotherm und einer adiabat betriebenen Reaktionszone.

Ein erfindungsgemäß ausgebildeter Reaktor 1 umfasst eine isotherme Reaktionszone 3, eine zweite isotherme Reaktionszone 5 und eine adiabate Reaktionszone 7. Die erste isotherme Reaktionszone 3 und die zweite isotherme Reaktionszone 5 sind jeweils als Strahlschlaufenreaktoren ausgebildet. Die erste isotherme Reaktionszone 3, die zweite isotherme Reaktionszone 5 und die adiabate Reaktionszone 7 sind in einer gemeinsamen Reaktorhülle 8 aufgenommen.

Das in der Zeichnung dargestellte Reaktorschema umfasst eine Durchflussregelung (FC), eine Durchflussverhältnis-Regelung (FFC), eine Druckregelung (PC) und eine Füllstandregelung (LC) als Regelelemente gemäß DIN EN 62424.

Die erste Reaktionszone 3 umfasst ein erstes Einsteckrohr 9, welches vom Reaktionsmedium durchströmt ist. Unterhalb des ersten Einsteckrohres 9 ist eine erste Prallplatte 11 positioniert. Flüssigkeit, die durch das erste Einsteckrohr 9 strömt, prallt auf die erste Prallplatte 11 und wird umgelenkt. Unterhalb der ersten Prallplatte 11 ist eine Flüssigkeitsentnahmestelle 13 vorgesehen, durch welche Reaktionsmedium aus der ersten isothermen Reaktionszone 3 entnommen werden kann.

Das an der Flüssigkeitsentnahmestelle 13 entnommene Reaktionsmedium wird einem ersten Flüssigkeitsumlauf 15 zugeführt. Im ersten Flüssigkeitsumlauf 15 ist eine erste Pumpe 17 aufgenommen, mit welcher die Flüssigkeit umgewälzt wird. In der hier dargestellten Ausführungsform schließt sich an die erste Pumpe 17 ein erster Wärmetauscher 19 an. Im ersten Wärmetauscher 19 wird das Reaktionsmedium, welches durch den ersten Flüssigkeitsumlauf 15 umgewälzt wird, temperiert. Hierdurch wird in der ersten isothermen Reaktionszone 3 eine gleichmäßige Temperatur erzielt. Über eine Flüssigkeitszugabevorrichtung 21 wird die Flüssigkeit aus dem ersten Flüssigkeitsumlauf 15 der ersten isothermen Reaktionszone 3 wieder zugeführt. Die Flüssigkeitszugabevorrichtung 21 ist vorzugsweise eine Düse. Die zugegebene Flüssigkeit wird mit hoher Geschwindigkeit in die erste Reaktionszone 3 eingespritzt. Hierdurch bildet sich eine Strömung durch das erste Einsteckrohr aus. Aufgrund der hohen Geschwindigkeit der über die Düse 21 eingespritzten Flüssigkeit wird auch die die Flüssigkeitszugabevorrichtung 21 umgebende Flüssigkeit mitgerissen. Es bildet sich eine Schlaufenströmung um das Einsteckrohr 9 aus. Die Umlenkung erfolgt dadurch, dass die Flüssigkeit, welche durch das Einsteckrohr 9 strömt, auf die Prallplatte 11 aufprallt und dort umgelenkt wird.

Die Zugabe des Aldehyds, welcher in dem Reaktor oxidiert werden soll, erfolgt über eine Flüssigkeitszugabe 23, welche in den ersten Flüssigkeitsumlauf 15 mündet. Die Menge an Aldehyd, welche über die Flüssigkeitszugabe 23 dem ersten Flüssigkeitsumlauf 15 zugeführt wird, lässt sich über ein hier dargestelltes Ventil 25 einstellen.

Die Flüssigkeitszugabevorrichtung 21 ist in der hier dargestellten Ausführungsform eine Zweistoffdüse. Über die Zweistoffdüse wird auch ein Teil des Sauerstoffes, welcher für die Reaktion benötigt wird zugegeben. Hierzu ist die Flüssigkeitszugabevorrichtung 21 mit einer Gaszufuhrleitung 27 verbunden. Über die Gaszufuhrleitung 27 wird Sauerstoff oder ein sauerstoffhaltiges Gas dem Reaktor 1 zugeführt. Das sauerstoffhaltige Gas ist zum Beispiel mit Sauerstoff angereicherte Luft oder reiner Sauerstoff. Bevorzugt enthält das sauerstoffhaltige Gas mindestens 50 Vol.-% Sauerstoff, mehr bevorzugt mindestens 75 Vol.-% und insbesondere mehr als 90 Vol.-% Sauerstoff. Ganz besonders bevorzugt ist das sauerstoffhaltige Gas reiner Sauerstoff, das heißt ein Gasgemisch mit einem Sauerstoffanteil von mehr als 99 Vol.-%.

Über die Gaszufuhrleitung 27 werden vorzugsweise mindestens 70 Vol.-%, mehr bevorzugt mindestens 80 Vol.-% des für die Reaktion benötigten Sauerstoffes zugeführt. Die Menge des zugeführten sauerstoffhaltigen Gases wird über ein zweites Ventil 29 eingestellt.

Ein Teil des in der ersten isothermen Reaktionszone 3 enthaltenen Reaktionsmediums strömt an einer zweiten Prallplatte 31 vorbei in die zweite isotherme Reaktionszone 5. Die zweite isotherme Reaktionszone 5 ist aufgebaut wie die erste isotherme Reaktionszone 3 und enthält ein zweites Einsteckrohr 33, welches vom Reaktionsmedium durchströmt wird. Das Reaktionsmedium prallt auf die zweite Prallplatte 31 und wird dort umgelenkt. Hierdurch ergibt sich eine Schlaufenströmung um das Einsteckrohr 33. Über eine zweite Flüssigkeitsentnahmestelle 35 wird ein Teil des Reaktionsmediums aus der zweiten isothermen Reaktionszone 5 entnommen und einem zweiten Flüssigkeitsumlauf 37 zugeführt. Die Umwälzung des Reaktionsmediums im zweiten Flüssigkeitsumlauf 37 erfolgt über eine zweite Pumpe 39. An die zweite Pumpe 39 schließt sich eine zweiter Wärmetauscher 41 an, in welchem das Reaktionsmedium, welches durch den zweiten Flüssigkeitsumlauf 37 strömt temperiert wird. Hierdurch wird die isotherme Reaktionsführung in der zweiten isothermen Reaktionszone 5 erzielt. Die Flüssigkeit, die den zweiten Flüssigkeitsumlauf 27 durchströmt, wird über eine zweite Flüssigkeitszugabevorrichtung 43 der zweiten isothermen Reaktionszone 5 zugeführt. Die zweite Flüssigkeitszugabevorrichtung 43 ist bevorzugt ebenfalls eine Zweistoffdüse. Über die als Zweistoffdüse ausgebildete zweite Flüssigkeitszugabevorrichtung 42 wird der restliche für die Reaktion benötigte Sauerstoff zugeführt. Hierzu ist die zweite Flüssigkeitszugabevorrichtung 43 mit einer zweiten Gaszufuhrleitung 45 verbunden. Die Menge des zugeführten Sauerstoffes über die zweite Gaszufuhrleitung 45 wird über ein drittes Ventil 47 eingestellt.

In der hier dargestellten Ausführungsform sind die erste Flüssigkeitszugabevorrichtung 21 und die zweite Flüssigkeitszugabevorrichtung 43 axial zentriert oberhalb des ersten Einsteckrohres 9 bzw. des zweiten Einsteckrohres 33 positioniert. Dies führt dazu, dass die Flüssigkeit jeweils im Einsteckrohr 9, 33 nach unten strömt und auf die Prallplatte 11, 31 auftrifft. Hier wird die Flüssigkeit umgelenkt und anschließend an der Außenseite des Einsteckrohres 9, 33 zwischen dem Einsteckrohr 9, 33 und der Reaktorhülle 8 nach oben transportiert.

Alternativ zu der hier dargestellten Ausführungsform ist es jedoch auch möglich, die erste Flüssigkeitszugabevorrichtung 21 und die zweite Flüssigkeitszugabevorrichtung 43 jeweils unterhalb des Einsteckrohres 9, 33 anzuordnen und die Prallplatte 11, 31 oberhalb des Einsteckrohres. In diesem Fall durchströmt die Flüssigkeit das Einsteckrohr von unten nach oben, wird an der Prallplatte 11, 31 umgeleitet und strömt außen zwischen Einsteckrohr 9, 33 und Kolonne 8 wieder nach unten.

Alternativ zu der hier dargestellten Ausführungsform, bei der der Wärmetauscher 19, 41 im Flüssigkeitsumlauf 15, 37 angeordnet ist, ist es auch möglich, einen ersten Wärmetauscher im Raum zwischen dem ersten Einsteckrohr 9 und der Reaktorhülle 8 und einen zweiten Wärmetauscher im Raum zwischen dem zweiten Einsteckrohr 33 und der Reaktorhülle 8 zu positionieren.

An die zweite isotherme Reaktionszone 5 schließt sich die adiabate Reaktionszone 7 an. Die adiabate Reaktionszone 7 enthält vorzugsweise Einbauten. In der hier dargestellten Ausführungsform sind dies Siebböden 49. Das Reaktionsmedium, welches in die adiabate Reaktionszone 7 strömt, enthält noch nicht umgesetzten Sauerstoff. Dieser liegt in Form von Blasen vor. Da in der adiabaten Reaktionszone 7 keine Umwälzung stattfindet, sondern eine Strömung von unten nach oben erfolgt, steigen die in dem Reaktionsmedium enthaltenen Gasblasen auf. Die adiabate Reaktionszone 7 wird somit in Form einer Blasensäule betrieben. Durch die in der adiabaten Reaktionszone 7 enthaltenen Siebböden 49 wird eine kaskadierte Blasensäule erzeugt.

Alternativ zu den Siebböden 49 können auch Lochböden oder beliebige andere, dem Fachmann bekannten Böden eingesetzt werden. Weiterhin ist es auch möglich als Einbauten eine Packung oder eine Schüttung vorzusehen.

An die adiabate Reaktionszone 7 schließt sich in der hier dargestellten Ausführungsform eine weitere Zone 51 an. Die weitere Zone 51 dient dazu, Gas und Flüssigkeit voneinander zu trennen. In der Zone 51 ist vorzugsweise eine Packung oder eine Schüttung enthalten. Diese dient dazu, die Folgen einer möglichen Explosion in der Gasphase zu mildern. Über einen Gasabzug 53 wird das überschüssige Gas aus dem Reaktor 1 abgezogen. Gleichzeitig kann über den Gasabzug 53 der Druck im Reaktor eingestellt werden.

Die Flüssigkeit wird über einen Seitenabzug 55 aus dem Reaktor abgezogen. Dieser befindet sich im Bereich der weiteren Zone 51. Während des Betriebes ist der gesamte Reaktor mit dem flüssigen Reaktionsmedium geflutet. Der Flüssigkeitsstand ist mit der Phasengrenzlinie 57 angedeutet.

Über ein Ventil 59 lässt sich die Menge der über den Seitenabzug 55 entnommenen Flüssigkeit einstellen. Gleichzeitig wird hierdurch der Flüssigkeitsstand im Reaktor geregelt.

### Beispiel 1

### Oxidation von Valeraldehyd zu Valeriansäure

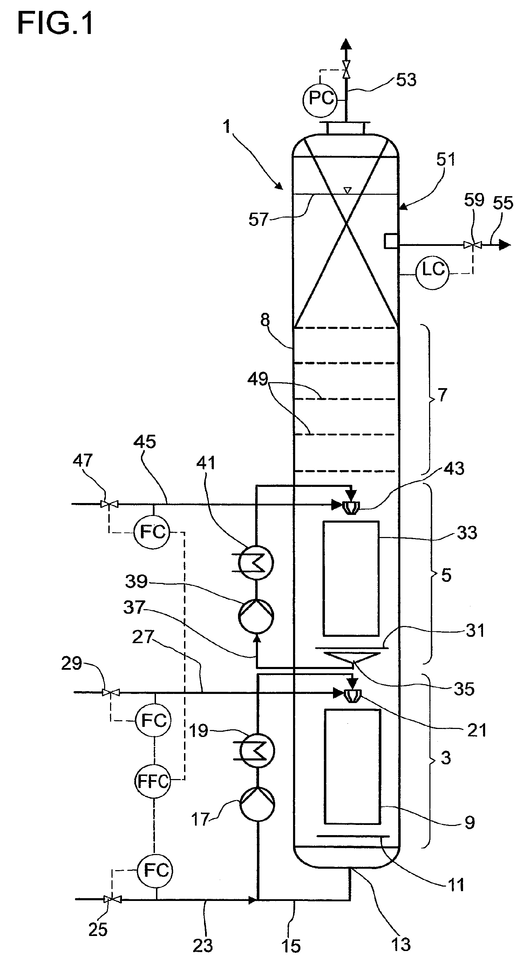

Es wird ein Reaktor, wie in Figur 1 dargestellt, zur Oxidation von Valeraldehyd zu Valeriansäure eingesetzt. Der Reaktor hat die Form eines aufrechten Zylinders mit etwa 940 mm Durchmesser und besteht aus zwei isothermen und einer adiabaten Stufe. Die erste isotherme Stufe, d.h. die erste von unten, hat eine Gesamtlänge, gemessen zwischen unterem Reaktorflansch und Zweistoffdüse der ersten Stufe, von etwa 5000 mm. Axial angeordnet befindet sich das Einsteckrohr mit 500 mm Durchmesser und 4100 mm Länge. Direkt oberhalb des Einsteckrohrs befindet sich axial zentriert und nach unten gerichtet die Zweistoffdüse der ersten Stufe. Unterhalb des Einsteckrohrs befindet sich eine axial zentrierte und waagerechte Prallplatte mit 620 mm Durchmesser. Über einen Bodenablass unterhalb der Prallplatte wird der Reaktorinhalt mit einer Umwälzpumpe angesaugt. An der Saugseite der Pumpe wird der frische Valeraldehyd zudosiert. An der Druckseite der Umwälzpumpe ist ein mit Kühlwasser betriebener shell-and-tube Wärmetauscher montiert, der dazu dient, die in der ersten Stufe erzeugte Reaktionswärme abzuführen. Der vom Wärmetauscher abgehende Strom wird zur Zweistoffdüse geleitet. Der in der ersten Stufe zur Oxidation benötigte Sauerstoff wird als Gas mit einem Sauerstoffanteil von >99% ebenfalls zur Zweistoffdüse geleitet. Diese Elemente bilden die erste isotherme Stufe des Reaktors.

Direkt oberhalb der ersten Stufe befindet sich die zweite isotherme Stufe. Diese hat im Wesentlichen die gleichen Elemente und Abmessungen wie die erste Stufe, jedoch wird der Reaktorinhalt der zweiten Stufe über einen Trichter unterhalb der Prallplatte der zweiten Stufe angesaugt, der Wärmetauscher und die Umwälzpumpe der zweiten Stufe sind entsprechend der geringeren Anforderungen kleiner dimensioniert, und die Zweistoffdüse der zweiten Stufe ist so dimensioniert, dass sie auch ohne Zugabe von Sauerstoff betrieben werden kann.

Oberhalb der zweiten isothermen Stufe befindet sich die adiabate Stufe. Diese Stufe besteht aus einem 5800 mm langen, leeren Rohr, das in Abständen von ca. 1400 mm durch Lochbleche unterteilt ist. Diese Lochbleche dienen einerseits dazu, Rückströmung zu verhindern und andererseits den noch vorhandenen Sauerstoff wieder in der Flüssigkeit zu verteilen.

Oberhalb der adiabaten Stufe befindet sich eine Beruhigungszone, in der sich Gas und Flüssigkeit trennen können. Aus Sicherheitsgründen ist die Beruhigungszone oder zumindest der Raum, der eine zusammenhängende Gasphase enthält, mit Raschig-Ringen mit einem Durchmesser von 10 mm gefüllt. Das Gas wird am Kopf des Reaktors druckgeregelt entnommen und zur Abgasbehandlung geleitet. Die Flüssigkeit wird standgeregelt über einen seitlichen Stutzen entnommen und zur Aufarbeitung geleitet.

Der Reaktor wird zunächst mit Valeriansäure gefüllt und die Umwälzpumpen werden in Betrieb genommen. Der Umwälzstrom in der ersten Stufe wird auf ca. 193 m³/h eingestellt. Die Druckdifferenz über die Umwälzpumpe beträgt ca. 4 bar. Der Umwälzstrom in der zweiten Stufe wird auf ca. 92 m³/h eingestellt. Die Druckdifferenz über die Umwälzpumpe der zweiten Stufe beträgt ca. 3 bar. Der Reaktorinhalt wurde dann auf 60°C erwärmt. Anschließend wird die Druckregelung am Reaktorkopf in Betrieb genommen und auf 2 bar abs eingestellt. Auch die standgeregelte Produktentnahme wird in Betrieb genommen. Danach wird die Dosierung von O₂ und Valeraldehyd schrittweise auf den Endwert angehoben. Im stationären Zustand werden 1125 kg/h Valeraldehyd zu dem Kreislauf der ersten isothermen Stufe zudosiert. Der eingesetzte Valeraldehyd stammt aus der Hydroformylierung von Buten und wird durch Destillation gereinigt. Der Anteil an 2-Methylbutanal beträgt weniger als 1 Gew.-%. Über die Zweistoffdüse der ersten Stufe werden 187 kg/h O₂ zudosiert. Über die Zweistoffdüse der zweiten Stufe werden 33 kg/h O₂ zudosiert.

Nach dem Erreichen des stationären Zustands werden Proben genommen. Der Valeraldehyd-Umsatz in der ersten isothermen Stufe beträgt 88,5, ermittelt durch gaschromatographische Analyse von Proben, die aus dem Umwälzstrom der ersten Stufe entnommen werden. Der Valeraldehyd-Umsatz in der zweiten isothermen Stufe beträgt 98,2%, ermittelt durch gaschromatographische Analyse von Proben, die aus dem Umwälzstrom der zweiten Stufe entnommen werden. Der Valeraldehyd-Umsatz am Reaktorausgang beträgt 99,94%. Die Selektivität von Valeriansäure bezogen auf Valeraldehyd beträgt 98,7 %, ermittelt durch gaschromatographische Analyse des flüssigen Produktaustrags.

Als Nebenprodukte sind im flüssigen Produkt u.a. n-Butylformiat, n-Butanol, Buttersäure, n-Octan, Laevulinsäure, Butylvalerat, Pentylvalerat und n-Valeriansäureanhydrid enthalten. Das Abgas enthält 0,69 Vol.-% organisches Material und ist demnach nicht zündfähig. Neben O₂ und Valeriansäure sind in der Gasphase noch CO₂, CO, H₂, Butan und Ameisensäure enthalten.

### Beispiel 2

### Oxidation von Valeraldehyd zu Valeriansäure

Es wird wie im Beispiel 1 verfahren, wobei 1125 kg/h Aldehyd zugegeben werden, mit dem Unterschied, dass die gesamte Menge von 220 kg/h O₂ der ersten isothermen Stufe zudosiert wird.

Der Valeraldehyd-Umsatz in der ersten isothermen Stufe beträgt 92,5 %. Der Valeraldehyd-Umsatz in der zweiten isothermen Stufe beträgt 98,8 %. Der Valeraldehyd-Umsatz am Reaktorausgang beträgt 99,93 %, ermittelt durch gaschromatographische Analyse des flüssigen Produktaustrags. Die Selektivität von Valeriansäure bezogen auf Valeraldehyd beträgt 98,7 %.

### Beispiel 3

### Oxidation von 3-Methylbutanal zu 3-Methylbuttersäure

Es wird wie im Beispiel 2 verfahren, d.h. es werden 1125 kg/h Aldehyd zugegeben und die gesamte Menge von 220 kg/h O₂ wird in der ersten Stufe zugegeben, aber anstatt Valeraldehyd wird 3-Methylbutanal eingesetzt. Das 3-Methylbutanal wird durch Hydroformylierung von Isobuten gewonnen und durch Destillation gereinigt. Es enthält mindestens 99 Gew.-% 3-Methylbutanal. Zum Anfahren wird der Reaktor mit 3-Methylbuttersäure statt mit Valeriansäure gefüllt.

Der 3-Methylbutanal-Umsatz in der ersten isothermen Stufe beträgt 97,4 %. Der 3-Methylbutanal-Umsatz in der zweiten isothermen Stufe beträgt 99,8 %. Der 3-Methylbutanal-Umsatz am Reaktorausgang beträgt 99,99 %. Die Selektivität von 3-Methylbuttersäure bezogen auf 3-Methylbutanal beträgt 97,1 %.

Als Nebenprodukte sind im flüssigen Produkt u.a. Isobutylformiat, Isobutanol, Isobuttersäure, 2,4-Dimethylpentan, 2,5-Dimethylhexan, 2-Butanon, Diisobutylether und 3-Methylbuttersäureanhydrid enthalten. Neben O₂ und 3-Methylbuttersäure sind in der Gasphase noch CO₂, CO, H₂, Isobutan, Isobuten, Acetaldehyd, Isopropylformiat, tert.-Butylformiat, Isobutylformiat und Ameisensäure enthalten.

### Beispiel 4

### Oxidation von Isononanal zu Isononansäure

Es wird wie im Beispiel 2 verfahren, d.h. es werden 1125 kg/h Aldehyd zugegeben und die gesamte Menge von 133 kg/h O₂ wird in der ersten Stufe zugeführt, aber anstatt Valeraldehyd wird Isononanal eingesetzt. Das Isononanal wird durch Hydroformylierung von Diisobutylen gewonnen und durch Destillation gereinigt. Es enthält mind. 99% C₉-Aldehyde mit 3,5,5-Trimethylhexanal als Hauptkomponente. Zum Anfahren wird der Reaktor mit Isononansäure statt mit Valeriansäure gefüllt.

Der Isononanal-Umsatz in der ersten isothermen Stufe beträgt 96,7 %. Der Isononanal-Umsatz in der zweiten isothermen Stufe beträgt 99,7 %. Der Isononanal-Umsatz am Reaktorausgang beträgt 99,98 %.

Als Nebenprodukte sind im flüssigen Produkt u.a. 2,4,4-Trimethylpentanolformiat, 2,4,4-Trimethylpentanol, Trimethylpentan und Isononylformiat enthalten. Das Abgas enthält 0,02 Vol.-% organisches Material und ist demnach nicht zündfähig. Neben O₂ und Isononansäure sind in der Gasphase noch CO₂, CO, H₂, 2,4,4-Trimethylpentan, u.a. enthalten.

### Beispiel 5

### Oxidation von Propionaldehyd zu Propionsäure

Es wird wie im Beispiel 2 verfahren, d.h. es werden 1125 kg/h Aldehyd zugegeben, die gesamte Menge von 326 kg/h O₂ wird in der ersten Stufe zugeführt, aber anstatt Valeraldehyd wird Propionaldehyd eingesetzt. Die Reaktionstemperatur in den beiden isothermen Stufen beträgt 75 °C. Der Propionaldehyd wird durch Hydroformylierung von Ethylen gewonnen und durch Destillation gereinigt. Zum Anfahren wird der Reaktor mit Propionsäure statt mit Valeriansäure gefüllt. Die Druckregelung am Reaktorkopf wird auf einen Druck von 2 bar abs eingestellt.

Der Propionaldehyd-Umsatz in der ersten isothermen Stufe beträgt 93,7 %. Der Propionaldehyd-Umsatz in der zweiten isothermen Stufe beträgt 98,5 %. Der Propionaldehyd-Umsatz am Reaktorausgang beträgt 99,8 %.

Als Nebenprodukte sind im flüssigen Produkt u.a. Ethylformiat, Ethanol, Essigsäure, enthalten. Neben O₂ und Propionsäure sind in der Gasphase noch CO₂, CO, H₂, Ethan, enthalten.

### Bezugszeichenliste

- 1: Reaktor
- 3: erste isotherme Reaktionszone
- 5: zweite isotherme Reaktionszone
- 7: adiabate Reaktionszone
- 8: Reaktorhülle
- 9: erstes Einsteckrohr
- 11: erste Prallplatte
- 13: Flüssigkeitsentnahmestelle
- 15: erster Flüssigkeitsumlauf
- 17: erste Pumpe
- 19: erster Wärmetauscher
- 21: Flüssigkeitszugabevorrichtung
- 23: Flüssigkeitszugabe
- 25: Ventil
- 27: Gaszufuhrleitung
- 29: zweites Ventil
- 31: zweite Prallplatte
- 33: zweites Einsteckrohr
- 35: zweite Flüssigkeitsentnahmestelle
- 37: zweiter Flüssigkeitsumlauf
- 39: zweite Pumpe
- 41: zweiter Wärmetauscher
- 43: zweite Flüssigkeitszugabevorrichtung
- 45: zweite Gaszufuhrleitung
- 47: drittes Ventil
- 49: Siebboden
- 51: weitere Zone
- 53: Gasabzug
- 55: Seitenabzug
- 57: Phasengrenzlinie
- 59: Ventil
- FC: Durchflussregelung
- FFC: Durchflussverhältnis-Regelung
- LC: Füllstandrege!ung
- PC: Druckregelung

## Patentansprüche

1. Verfahren zur Oxidation von Aldehyden mit Sauerstoff, welches folgende Schritte aufweist:
(a) Zugabe des Aldehyds und zumindest eines Teils des für die Oxidation benötigten Sauerstoffs in eine erste Reaktionszone, wobei die erste Reaktionszone isotherm und rückvermischt betrieben wird,
(b) Zufuhr zumindest eines Teils des Reaktionsgemisches aus der ersten Reaktionszone in eine zweite Reaktionszone, wobei die zweite Reaktionszone adiabat betrieben wird
und wobei alle Reaktionszonen in einer gemeinsamen Reaktorhülle aufgenommen sind.

2. Verfahren nach Anspruch 1, **dadurch gekennzeichnet, dass** zwischen der ersten Reaktionszone und der zweiten Reaktionszone mindestens eine weitere isotherm und rückvermischt betriebene Reaktionszone ausgebildet ist, wobei vorzugsweise der für die Reaktion erforderliche Teil des Sauerstoffs, der nicht in Schritt (a) zugegeben worden ist, dem Reaktionsgemisch in der mindestens einen weiteren isotherm und rückvermischt betriebenen Reaktionszone zugegeben wird.

3. Verfahren nach Anspruch 1 oder 2, **dadurch gekennzeichnet, dass** die mindestens eine isotherm und rückvermischt betriebene Reaktionszone nach dem Prinzip eines Schlaufenreaktors arbeitet, wobei ein Teil des Reaktionsgemisches der Reaktionszone entnommen wird und über eine Düse im oberen Bereich der Reaktionszone wieder zugeführt wird, wodurch eine Ringströmung in der Reaktionszone erzeugt wird, wobei der Teil des Reaktionsgemisches, der der isotherm und rückvermischt betriebenen Reaktionszone entnommen und über die Düse im oberen Bereich der Reaktionszone wieder zugeführt wird vorzugsweise durch einen Wärmetauscher geführt wird, um eine isotherme Temperaturführung in der Reaktionszone zu realisieren.

4. Verfahren nach einem der Ansprüche 1 bis 3, **dadurch gekennzeichnet, dass** die adiabat betriebene Reaktionszone in Form einer Blasensäule ausgebildet ist, wobei in der adiabat betriebenen Reaktionszone vorzugsweise mindestens ein Siebboden oder Lochblech enthalten ist, durch den der Durchfluss des Reaktionsgemisches durch die adiabat betriebene Reaktionszone eingestellt wird.

5. Verfahren nach einem der Ansprüche 1 bis 4, **dadurch gekennzeichnet, dass** der Aldehyd, der zu seiner korrespondierenden Säure oxidiert wird, ausgewählt ist aus Propionaldehyd, Butyraldehyd, Isobutyraldehyd, Valeraldehyd, 2-Methylbutyraldehyd, Isovaleraldehyd, 2-Ethylbutyraldehyd, n-Hexanal, n-Heptanal, 2-Ethylhexanal, 2-Propylheptanal, 3,5,5-Trimethylhexanal und 3,7-Dimethyloctanal.

6. Reaktor zur Durchführung des Verfahrens nach einem der Ansprüche 1 bis 5, umfassend mindestens eine isotherme (3) und eine adiabate (7) Reaktionszone, die in einer Reaktorhülle (8) angeordnet sind, wobei jede isotherme Reaktionszone (3) in Form eines Strahlschlaufenreaktors ausgebildet ist und die adiabate Reaktionszone (7) als Blasensäule ausgebildet ist.

7. Reaktor nach Anspruch 6, **dadurch gekennzeichnet, dass** in der Reaktorhülle (8) zwei isotherme Reaktionszonen (3, 5) enthalten sind, die in Form eines Strahlschlaufenreaktors ausgebildet sind, wobei die beiden in Form eines Strahlschlaufenreaktors ausgebildeten isothermen Reaktionszonen (3, 5) übereinander in der Reaktorhülle (8) angeordnet sind.

8. Reaktor nach Anspruch 6 oder 7, **dadurch gekennzeichnet, dass** jeweils im unteren Bereich der einzelnen in Form eines Strahlschlaufenreaktors ausgebildeten isothermen Reaktionszonen (3, 5) eine Prallplatte (11, 31) aufgenommen ist.

9. Reaktor nach einem der Ansprüche 6 bis 8, **dadurch gekennzeichnet, dass** die mindestens eine als Strahlschlaufenreaktor ausgebildete isotherme Reaktionszone (3, 5) einen äußeren Flüssigkeitsumlauf (15, 37) aufweist, wobei eine Flüssigkeitsentnahmestelle (13, 35) im unteren Bereich der isothermen Reaktionszone (3, 5) und eine Flüssigkeitszugabevorrichtung (21, 43) im oberen Bereich der isothermen Reaktionszone (3, 5) angeordnet ist, wobei die Flüssigkeitszugabevorrichtung (21, 43) vorzugsweise eine Düse ist, die zentral im oberen Bereich der isothermen Reaktionszone (3, 5) angeordnet ist und die Düse vorzugsweise eine Zweistoffdüse ist, über welche Flüssigkeit und Sauerstoff in die isotherme Reaktionszone (3, 5) zugegeben werden können.

10. Reaktor nach Anspruch 9, **dadurch gekennzeichnet, dass** im äußeren Flüssigkeitsumlauf (15, 37) ein Wärmetauscher (19, 41) enthalten ist oder im äußeren Bereich der in Form eines Strahlschlaufenreaktors ausgebildeten isothermen Reaktionszone (3, 5) zwischen einem Einsteckrohr (9, 33) und der Reaktorhülle (8) ein Wärmetauscher aufgenommen ist.

11. Reaktor nach Anspruch 9 oder 10, **dadurch gekennzeichnet, dass** in den Flüssigkeitsumlauf (15) der ersten isothermen Reaktionszone (3) eine Flüssigkeitszugabe (23) für ein Edukt mündet.

12. Reaktor nach einem der Ansprüche 6 bis 11, **dadurch gekennzeichnet, dass** in der adiabaten Reaktionszone (7) Lochbleche oder Siebböden (49) enthalten sind.

13. Reaktor nach einem der Ansprüche 6 bis 12, **dadurch gekennzeichnet, dass** sich an die adiabate Reaktionszone (7) eine weitere Zone (51) zur Phasentrennung anschließt, wobei die weitere Zone (51) eine Schüttung oder Packung enthält.

14. Reaktor nach einem der Ansprüche 6 bis 13, **dadurch gekennzeichnet, dass** die Reaktionszonen (3, 5, 7) übereinander angeordnet sind, wobei die erste isotherme Reaktionszone (3) unten und die adiabate Reaktionszone (7) oben in der Reaktorhülle (8) angeordnet sind.

15. Verwendung des Reaktors nach einem der Ansprüche 6 bis 14 zur Herstellung einer organischen Säure durch Oxidation des korrespondierenden Aldehyds mit Sauerstoff, wobei der Aldehyd vorzugsweise ausgewählt ist aus Propionaldehyd, Butyraldehyd, Isobutyraldehyd, Valeraldehyd, 2-Methylbutyraldehyd, Isovaleraldehyd, 2-Ethylbutyraldehyd, n-Hexanal, n-Heptanal, 2-Ethylhexanal, 2-Propylheptanal, 3,5,5-Trimethylhexanal und 3,7-Dimethyloctanal.

## Claims

1. A process for the oxidation of aldehydes by means of oxygen, which comprises the following steps:
(a) introduction of the aldehyde and at least part of the oxygen required for the oxidation into a first reaction zone which is operated isothermally and with backmixing,
(b) introduction of at least part of the reaction mixture from the first reaction zone into a second reaction zone which is operated adiabatically, where all reaction zones are accommodated in a common reactor shell.

2. The process according to claim 1, wherein at least one further reaction zone which is operated isothermally and with backmixing is configured between the first reaction zone and the second reaction zone, and the part of the oxygen required for the reaction which has not been introduced in step (a) is preferably introduced into the reaction mixture in the at least one further reaction zone which is operated isothermally and with backmixing.

3. The process according to claim 1 or 2, wherein the at least one reaction zone which is operated isothermally and with backmixing operates according to the principle of a loop reactor, with part of the reaction mixture being taken off from the reaction zone and reintroduced via a nozzle in the upper region of the reaction zone, thus producing circular flow in the reaction zone, where the part of the reaction mixture which is taken off from the reaction zone which is operated isothermally and with backmixing and reintroduced via the nozzle in the upper region of the reaction zone is preferably conveyed through a heat exchanger in order to achieve isothermal conditions in the reaction zone.

4. The process according to any of claims 1 to 3, wherein the adiabatically operated reaction zone is configured in the form of a bubble column, where the adiabatically operated reaction zone preferably comprises at least one sieve tray or perforated plate by means of which the flow of the reaction mixture through the adiabatically operated reaction zone is set.

5. The process according to any of claims 1 to 4, wherein the aldehyde, which is oxidized to its corresponding acid, is selected from among propionaldehyde, butyraldehyde, isobutyraldehyde, valeraldehyde, 2-methylbutyraldehyde, isovaleraldehyde, 2-ethylbutyraldehyde, n-hexanal, n-heptanal, 2-ethylhexanal, 2-propylheptanal, 3,5,5-trimethylhexanal and 3,7-dimethyloctanal.

6. A reactor for carrying out the process according to any of claims 1 to 5, which comprises at least one isothermal reaction zone (3) and an adiabatic reaction zone (7) which are arranged in a reactor shell (8), wherein each isothermal reaction zone (3) is configured in the form of a jet loop reactor and the adiabatic reaction zone (7) is configured as a bubble column.

7. The reactor according to claim 6, wherein the reactor shell (8) comprises two isothermal reaction zones (3, 5) which are configured in the form of a jet loop reactor and are arranged above one another in the reactor shell (8).

8. The reactor according to claim 6 or 7, wherein an impingement plate (11, 31) is accommodated in the lower region of each of the individual isothermal reaction zones (3, 5) configured in the form of a jet loop reactor.

9. The reactor according to any of claims 6 to 8, wherein the at least one isothermal reaction zone (3, 5) configured as a jet loop reactor has an external liquid circuit (15, 37) and a liquid offtake point (13, 35) is located in the lower region of the isothermal reaction zone (3, 5) and a liquid introduction device (21, 43) is located in the upper region of the isothermal reaction zone (3, 5), where the liquid introduction device (21, 43) is preferably a nozzle which is located centrally in the upper region of the isothermal reaction zone (3, 5) and the nozzle is preferably a two-fluid nozzle via which liquid and oxygen can be introduced into the isothermal reaction zone (3, 5).

10. The reactor according to claim 9, wherein a heat exchanger (19, 41) is comprised in the external liquid circuit (15, 37) or a heat exchanger is accommodated in the outer region of the isothermal reaction zone (3, 5) configured in the form of a jet loop reactor between a plug-in tube (9, 33) and the reactor shell (8).

11. The reactor according to claim 9 or 10, wherein a liquid inlet (23) for a starting material opens into the liquid circuit (15) of the first isothermal reaction zone (3).

12. The reactor according to any of claims 6 to 11, wherein perforated plates or sieve trays (49) are comprised in the adiabatic reaction zone (7).

13. The reactor according to any of claims 6 to 12, wherein the adiabatic reaction zone (7) is followed by a further zone (51) for phase separation, with the further zone (51) comprising a bed or ordered packing.

14. The reactor according to any of claims 6 to 13, wherein the reaction zones (3, 5, 7) are arranged above one another, with the first isothermal reaction zone (3) being located at the bottom and the adiabatic reaction zone (7) being located at the top in the reactor shell (8).

15. The use of the reactor according to any of claims 6 to 14 for preparing an organic acid by oxidation of the corresponding aldehyde by means of oxygen, where the aldehyde is preferably selected from among propionaldehyde, butyraldehyde, isobutyraldehyde, valeraldehyde, 2-methylbutyraldehyde, isovaleraldehyde, 2-ethylbutyraldehyde, n-hexanal, n-heptanal, 2-ethylhexanal, 2-propylheptanal, 3,5,5-trimethylhexanal and 3,7-dimethyloctanal.

## Revendications

1. Procédé d'oxydation d'aldéhydes avec de l'oxygène, qui comprend les étapes suivantes :
(a) l'ajout de l'aldéhyde et d'au moins une partie de l'oxygène nécessaire pour l'oxydation dans une première zone de réaction, la première zone de réaction étant exploitée sous forme isotherme et rétromélangée,
(b) l'introduction d'au moins une partie du mélange réactionnel de la première zone de réaction dans une seconde zone de réaction, la seconde zone de réaction étant exploitée sous forme adiabatique,
et toutes les zones de réaction étant logées dans une enveloppe de réacteur commune.

2. Procédé selon la revendication 1, **caractérisé en ce qu'**au moins une autre zone de réaction exploitée sous forme isotherme et rétromélangée est formée entre la première zone de réaction et la seconde zone de réaction, la partie de l'oxygène nécessaire pour la réaction qui n'a pas été ajoutée à l'étape (a) étant de préférence ajoutée au mélange réactionnel dans ladite au moins une autre zone de réaction exploitée sous forme isotherme et rétromélangée.

3. Procédé selon la revendication 1 ou 2, **caractérisé en ce que** ladite au moins une zone de réaction exploitée sous forme isotherme et rétromélangée fonctionne selon le principe d'un réacteur à boucle, une partie du mélange réactionnel étant extraite de la zone de réaction et réintroduite par une buse dans la partie supérieure de la zone de réaction, un écoulement circulaire dans la zone de réaction étant ainsi généré, la partie du mélange réactionnel qui est extraite de la zone de réaction exploitée sous forme isotherme et rétromélangée et réintroduite par une buse dans la partie supérieure de la zone de réaction traversant de préférence un échangeur de chaleur afin de réaliser une commande de température isotherme dans la zone de réaction.

4. Procédé selon l'une quelconque des revendications 1 à 3, **caractérisé en ce que** la zone de réaction exploitée sous forme adiabatique est configurée sous la forme d'une colonne à bulles, au moins un plateau perforé ou une tôle perforée étant de préférence contenu dans la zone de réaction exploitée sous la forme adiabatique, par lequel le passage du mélange réactionnel dans la zone de réaction exploitée sous forme adiabatique est ajusté.

5. Procédé selon l'une quelconque des revendications 1 à 4, **caractérisé en ce que** l'aldéhyde qui est oxydé en son acide correspondant est choisi parmi le propionaldéhyde, le butyraldéhyde, l'isobutyraldéhyde, le valéraldéhyde, le 2-méthylbutyraldéhyde, l'isovaléraldéhyde, le 2-éthylbutyraldéhyde, le n-hexanal, le n-heptanal, le 2-éthylhexanal, le 2-propylheptanal, le 3,5,5-triméthylhexanal et le 3,7-diméthyloctanal.

6. Réacteur pour la réalisation du procédé selon l'une quelconque des revendications 1 à 5, comprenant au moins une zone de réaction isotherme (3) et une zone de réaction adiabatique (7) qui sont agencées dans une enveloppe de réacteur (8), chaque zone de réaction isotherme (3) étant configurée sous la forme d'un réacteur à boucle et à jet et la zone de réaction adiabatique (7) étant configurée sous la forme d'une colonne à bulles.

7. Réacteur selon la revendication 6, **caractérisé en ce que** deux zones de réaction isothermes (3, 5) sont contenues dans l'enveloppe de réacteur (8), qui sont configurées sous la forme d'un réacteur à boucle et à jet, les deux zones de réaction isothermes configurées sous la forme d'un réacteur à boucle et à jet (3, 5) étant agencées l'une sur l'autre dans l'enveloppe de réacteur (8).

8. Réacteur selon la revendication 6 ou 7, **caractérisé en ce qu'**un déflecteur (11, 31) est logé à chaque fois dans la partie inférieure des zones de réaction isothermes configurées sous la forme d'un réacteur à boucle et à jet (3, 5) individuelles.

9. Réacteur selon l'une quelconque des revendications 6 à 8, **caractérisé en ce que** ladite au moins une zone de réaction isotherme configurée sous la forme d'un réacteur à boucle et à jet (3, 5) comprend un circuit de liquide extérieur (15, 37), un emplacement de soutirage de liquide (13, 35) étant agencé dans la partie inférieure de la zone de réaction isotherme (3, 5) et un dispositif d'ajout de liquide (21, 43) dans la partie supérieure de la zone de réaction isotherme (3, 5), le dispositif d'ajout de liquide (21, 43) étant de préférence une buse qui est agencée au centre dans la partie supérieure de la zone de réaction isotherme (3, 5) et la buse étant de préférence une buse à deux composants, par le biais de laquelle un liquide et de l'oxygène peuvent être ajouté dans la zone de réaction isotherme (3, 5).

10. Réacteur selon la revendication 9, **caractérisé en ce qu'**un échangeur de chaleur (19, 41) est contenu dans le circuit de liquide extérieur (15, 37) ou un échangeur de chaleur est logé dans la partie extérieure de la zone de réaction isotherme configurée sous la forme d'un réacteur à boucle et à jet (3, 5) entre un tube d'insertion (9, 33) et l'enveloppe du réacteur (8).

11. Réacteur selon la revendication 9 ou 10, **caractérisé en ce qu'**un dispositif d'ajout de liquide (23) pour un réactif débouche dans le circuit de liquide (15) de la première zone de réaction isotherme (3).

12. Réacteur selon l'une quelconque des revendications 6 à 11, **caractérisé en ce que** des tôles perforées ou des plateaux perforés (49) sont contenus dans la zone de réaction adiabatique (7).

13. Réacteur selon l'une quelconque des revendications 6 à 12, **caractérisé en ce qu'**une zone supplémentaire (51) pour la séparation de phases suit la zone de réaction adiabatique (7), la zone supplémentaire (51) contenant un remplissage ou un garnissage.

14. Réacteur selon l'une quelconque des revendications 6 à 13, **caractérisé en ce que** les zones de réaction (3, 5, 7) sont agencées les unes sur les autres, la première zone de réaction isotherme (3) étant agencée en dessous et la zone de réaction adiabatique (7) au-dessus dans l'enveloppe du réacteur (8).

15. Utilisation du réacteur selon l'une quelconque des revendications 6 à 14 pour la fabrication d'un acide organique par oxydation de l'aldéhyde correspondant avec de l'oxygène, l'aldéhyde étant de préférence choisi parmi le propionaldéhyde, le butyraldéhyde, l'isobutyraldéhyde, le valéraldéhyde, le 2-méthylbutyraldéhyde, l'isovaléraldéhyde, le 2-éthylbutyraldéhyde, le n-hexanal, le n-heptanal, le 2-éthylhexanal, le 2-propylheptanal, le 3,5,5-triméthylhexanal et le 3,7-diméthyloctanal.
